# EUROPÄISCHE PATENTSCHRIFT

(11) **EP 2 066 397 B1**
(45) Veröffentlichungstag und Bekanntmachung des Hinweises auf die Patenterteilung: **17.02.2010**
(21) Anmeldenummer: 07800155.9
(22) Anmeldetag: 24.08.2007
(51) Int. Cl.: A61N 1/05

(54) **MEHRKANALELEKTRODE FÜR COCHLEA-IMPLANTATE MIT EINER MEHRZAHL VON ÜBER DIE LÄNGE DER ELEKTRODE VERTEILTEN KONTAKTEN**
MULTI-CHANNEL ELECTRODE FOR COCHLEA IMPLANTS, WITH A PLURALITY OF CONTACTS DISTRIBUTED ALONG THE LENGTH OF THE ELECTRODE
ÉLECTRODE MULTICANAL POUR IMPLANTS COCHLÉAIRES AVEC UNE PLURALITÉ DE CONTACTS RÉPARTIS SUR LA LONGUEUR DE L'ÉLECTRODE

(30) Priorität: 24.08.2006 AT 14152006
(43) Veröffentlichungstag der Anmeldung: 10.06.2009
(73) Patentinhaber: Med-El Elektromedizinische Geräte Gesellschaft m.b.H., 6020 Innsbruck (AT)
(72) Erfinder: HAMZAVI, J., Sasan, A-1090 Wien (AT); ARNOLDNER, Christoph, A-1130 Wien (AT)
(74) Vertreter: Lucke, Andreas
(86) Internationale Anmeldenummer: PCT/AT2007/000405
(87) Internationale Veröffentlichungsnummer: WO 2008/022366

(56) Entgegenhaltungen:
- WO-A-97/30670
- US-B1- 6 421 569
- US-B2- 6 565 503

## Beschreibung

Die Erfindung bezieht sich auf eine Mehrkanalelektrode für Cochlea-Implantate mit einer Mehrzahl von über die Länge der Elektrode verteilten Kontakten.

Cochlea-Implantate dienen dazu, über Mikrophone erzeugte elektrische Signale in einer Weise zu transformieren, dass in der Folge über in die Schnecke bzw. Cochlea eingebrachte Elektroden eine entsprechende Nervenreizung ermöglicht wird, welche die entsprechende Reizübertragung in das Hörzentrum des Gehirns ermöglichen. Derartige Cochlea-Implantate umgehen hierbei die mechanische Übertragung vom Schall auf das neuronale System und erlauben es Nerven bzw. Nervenenden unmittelbar elektrisch zu stimulieren, um auf diese Weise den beim gesunden Ohr funktionierenden mechanischen Stimulus zur Auslösung von Reizsignalen zu substituieren. In diesem Zusammenhang wird ein hoher elektronischer Aufwand getrieben und ein komplexer Algorithmus verwendet, wobei die elektronisch generierten Signale an Mehrkanalelektroden geführt werden, und die Signale in aller Regel zyklisch an die einzelnen Kanäle der Mehrkanalelektrode angelegt werden.

Die Elektroden selbst werden bei bekannten Cochlea-Implantaten in die Schnecke eingeschoben, wobei hier entsprechend der Bauform der Elektrode eine bestimmte Insertionstiefe erreichbar ist. Konventionelle Mehrkanalelektroden weisen hierbei 12 oder mehr Kontakte auf, wobei derartige Elektroden nur über eine Insertionstiefe von üblicherweise 17 bis 25 mm einschiebbar sind. Die Cochlea stellt ein bereits sehr früh im menschlichen Körper vollständig ausgebildetes Organ dar, dessen absolute Größe sich beim Neugeborenen nicht wesentlich von der Größe bei einem erwachsenen Menschen unterscheidet. Cochlea-Implantate können nun prinzipiell bei gehörlosen Neugeborenen mit Erfolg eingesetzt werden, welche mittels eines Cochlea-Implantats die Sprache erlernen können oder aber bei erwachsenen Menschen, bei welchen nach dem Erlernen der Sprache und nach einer bestimmten Zeit, über welche das Gehör intakt funktionierte, ein mechanischer Defekt eingetreten ist, welcher das Hörvermögen reduziert oder zunichte gemacht hat. In diesen Fällen gelingt es, mit den bekannten elektronischen Algorithmen eine elektrische Ersatzstimulation vorzunehmen, welche insgesamt zur Sprachverständlichkeit führt.

Die Begrenzung der Einschubtiefe wurde in der Literatur in der Regel damit begründet, dass die Cochlea nur im basalen und mittleren Bereich über hinreichend viele Nervenenden verfügt, deren Reizung bzw. Stimulation zu sinnvollen Auswertungen im Hörzentrum führen kann. Die Insertionstiefe der Elektroden wurde aus diesen Gründen auf den basalen und mittleren Bereich beschränkt, wobei nur vereinzelt auch längere Elektroden am Markt erschienen sind, welche prinzipiell auch bis in die apikale Region des kortischen Organs vordringen könnten. Eine Zuordnung der Elektrodenposition zur relativen Lage im inneren des kortischen Organs wurde insoweit bereits versucht als festgestellt werden konnte, dass die basale Region für die Aufnahme von Frequenzen von 1 kHz und 8 bis 10 kHz besonders geeignet ist, wohingegen die nach innen nachfolgende mittlere Region den Bereich von 1 kHz und tiefer bevorzugt erfasst. Weiter innen liegende Regionen können in der Folge auch niedere Frequenzen abtasten.

Bekannte Mehrkanalelektroden für Cochlea-Implantate zeichnen sich durch eine maximale Länge bis 32 mm aus, wobei die Abstände der Elektroden über die Insertionstiefe konstant gehalten wurden.

Aus der US 7076308 B1 ist eine herkömmliche Mehrkanalelektrode bekannt geworden, wobei hier ein verbessertes Verfahren zum Ermitteln und Einstellen der jeweils erforderlichen Stimulationsströme beschrieben ist.

Die Patentschrift US-6 565 503 B2 offenbart ein Cochfear-Implantat bei dem die Elektroden entweder in konstantem Abstand auf einem Träger angeordnet sind oder in logarithmischem, um so besser den Frequenzverhältnissen entlang der Basilarmembran Rechnung zu tragen.

Die US 7039466 B1 beschreibt ein Cochlea-Implantat, bei welchem der apikale Bereich der Cochlea mit einer verminderten Stimulationsrate stimuliert wird als der basale Bereich.

Eine Mehrkanalelektrode für ein Cochlea-Implantat ist ebenso aus der US 2006/136030 A1 bekannt geworden.

Die Erfindung zielt nun darauf ab, eine Mehrkanalelektrode der eingangs genannten Art zu schaffen, mit welcher nicht nur die akustische Wahrnehmung sondern vor allen Dingen die Sprachverständlichkeit weiter verbessert wird und unerwünschte Störungen durch Übersprechen benachbarter Kanäle weiter verhindert werden. Zur Lösung dieser Aufgabe besteht die erfindungsgemäße Mehrkanalelektrode der eingangs genannten Art im Wesentlichen darin, dass der Abstand der aktiven Kontakte voneinander in einem apikalen Endbereich größer gewählt ist, als in einem basalen Bereich. Überraschenderweise hat sich bei einer derartigen Ausbildung gezeigt, dass im apikalen Endbereich nicht etwa nur tiefe Frequenzen besser erfasst werden, sondern dass bei entsprechend größeren Abständen von aktiven Kontakten im apikalen Endbereich und damit in einer Insertionstiefe, die in der Regel größer ist als die Insertionstiefe bekannter Mehrkanalelektroden, die Sprachverständlichkeit verbessert wird. Dies ist umso überraschender als in der Literatur das apikale Ende im Inneren der Cochlea als weniger empfindlich beschrieben wird und tiefe Frequenzen somit nicht notwendigerweise die Sprachverständlichkeit verbessern. Die Vergrößerung der Abstände der aktiven Kontakte im apikalen Bereich führte aber nun zum überraschenden Ergebnis, dass bei insgesamt relativ geringer Anzahl von Kanälen ein erhöhtes Maß an Sprachverständlichkeit erzielt werden kann.

Mit Vorteil ist die erfindungsgemäße Ausbildung so getroffen, dass der Bereich mit größerem Kontaktabstand einer Insertionstiefe der Elektrode von über 20 mm, vorzugsweise 20 bis 32 mm entspricht, womit sichergestellt wird, dass die Mehrkanalelektrode tatsächlich mit ihrem apikalen Endbereich bis weit in das Ende der Schnecke hinein eingeschoben werden kann. Die Ausbildung kann hierbei bevorzugt so getroffen sein, dass der Kontaktabstand in wenigstens zwei Abstandstufen vergrößert ist, wobei in der ersten, einem mittleren Bereich der Elektrode entsprechenden Teilbereich der Kontaktabstand um 25 bis 50%, insbesondere etwa ⅓ vergrößert ist und im apikalen Ende dem 1,5-fachen bis 2,5-fachen, insbesondere dem 2-fachen des zuvor vergrößerten Kontaktabstandes entspricht, wobei vorzugsweise der Abstand der Kontakte im apikalen Bereich wenigstens dem 2,5-fachen des Abstandes der Kontakte im basalen Bereich der Elektrode entspricht. Eine derartige Bemessung hat bei gleichzeitig relativ geringer Anzahl von Kontakten ein besonders hohes Maß an Sprachverständlichkeit ergeben. Die Testelektroden verfügen hierbei über 12 und weniger Kontakte, wohingegen übliche Elektrode bekannter Bauart, welche Insertionstiefen von 16 bis 22 mm erreichen, eine weit größere Anzahl von Kontakten aufweisen. Die Verringerung der Anzahl der Kontakte führt hierbei gleichzeitig zu einer weiteren Verringerung des Kanalübersprechens.

Eine besonders vorteilhafte Ausbildung mit besonders guter Sprachverständlichkeit kann dann erzielt werden, wenn die Ausbildung so getroffen ist, dass die Kontaktabstände vom basalen Ende zum apikalen Ende im Übergangsbereich zwischen basalem und mittlerem Bereich zunächst abnehmen und im mittleren und apikalen Bereich zunehmen.

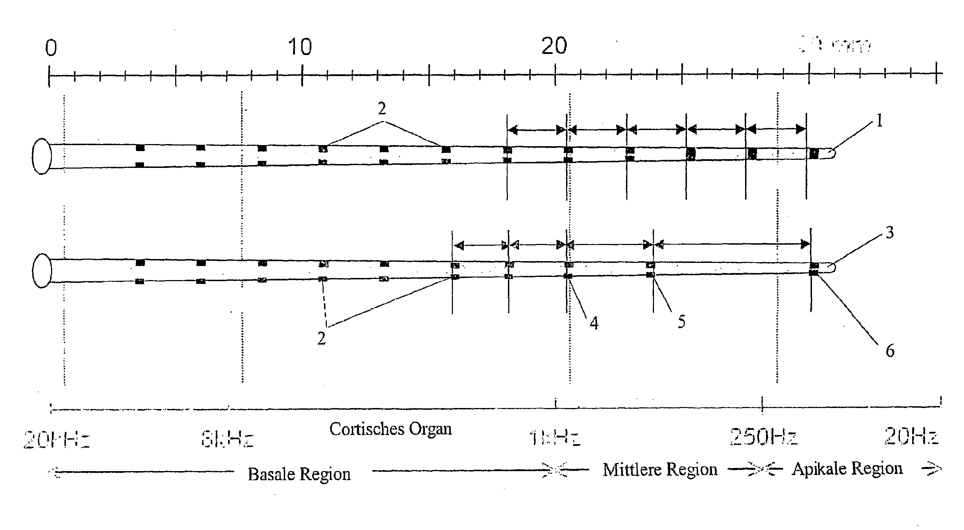

Die Erfindung wird nachfolgend anhand von in der Zeichnung dargestellten Ausbildungen einer Standardelektrode und einer erfindungsgemäß modifizierten Elektrode näher erläutert. In der Zeichnung ist mit 1 eine Standardelektrode mit 12 Kontakten dargestellt, wobei der Abstand der Kontakte zueinander im Wesentlichen konstant gehalten ist. Die Kontakte sind hierbei mit 2 bezeichnet und jeweils gesondert über gesonderte Leitungen mit der zugehörigen Steuerelektronik verbunden. Die mit 3 bezeichnete erfindungsgemäße Elektrode weist in der basalen Region weitestgehend konstante Kontaktabstände auf. Dieser Bereich des kortischen Organs wird üblicherweise für die Erkennung von Frequenzen zwischen 15 kHz und 1 kHz verantwortlich gemacht. In der nachfolgenden mittleren Region, welche einer Einschubtiefe von über 20 mm entspricht, wird der entsprechende Kontaktabstand um einen Faktor 1,33 vergrößert, wobei die entsprechenden Kontakte nunmehr mit 4 und 5 bezeichnet sind. Der Abstand zwischen dem Kontakt 5 und dem Kontakt 6 im apikalen Ende der Elektrode 3 entspricht nun etwa dem doppelten dieses Abstandes der Kontakte 4 und 5 und reicht bis weit in den apikalen Bereich hinein und liegt somit bei einer Insertionstiefe von etwa 30 mm. In der Zeichnung ist hierbei jeweils die entsprechende Insertionstiefe am oberen Rand eingetragen. Die entsprechende Frequenzempfindlichkeit des kortischen Organs sowie die einzelnen Regionen, nämlich die basale, die mittlere und die apikale Region, entsprechend der jeweiligen Insertionstiefe, sind am unteren Rand der Zeichnung dargestellt.

## Patentansprüche

1. Mehrkanalelektrode für Cochlea-Implantate mit einer Mehrzahl von über die Länge der Elektrode verteilten Kontakten, **dadurch gekennzeichnet, dass** der Abstand der aktiven Kontakte (2,4,5, 6) voneinander in einem apikalen Endbereich größer gewählt ist, als in einem basalen Bereich.

2. Mehrkanalelektrode nach Anspruch 1, **dadurch gekennzeichnet, dass** der Bereich mit größerem Kontaktabstand einer Insertionstiefe der Elektrode (3) von über 20 mm, vorzugsweise 20 bis 32 mm entspricht.

3. Mehrkanalelektrode nach Anspruch 1 oder 2, **dadurch gekennzeichnet, dass** der Kontaktabstand in wenigstens zwei Abstandstufen vergrößert ist, wobei in der ersten, einem mittleren Bereich der Elektrode (3) entsprechenden Teilbereich der Kontaktabstand um 25 bis 50%, insbesondere etwa ⅓ vergrößert ist und im apikalen Ende dem 1,5-fachen bis 2,5-fachen, insbesondere dem 2-fachen des zuvor vergrößerten Kontaktabstandes entspricht.

4. Mehrkanalelektrode nach Anspruch 1, 2 oder 3, **dadurch gekennzeichnet, dass** der Abstand der Kontakte im apikalen Bereich wenigstens dem 2,5-fachen des Abstandes der Kontakte im basalen Bereich der Elektrode (3) entspricht.

5. Mehrkanalelektrode nach einem der Ansprüche 1 bis 4, **dadurch gekennzeichnet, dass** die Kontaktabstände vom basalen Ende zum apikalen Ende im Übergangsbereich zwischen basalem und mittlerem Bereich zunächst abnehmen und im mittleren und apikalen Bereich zunehmen.

## Claims

1. Multi-channel electrode for cochlear implants, having a plurality of contacts distributed over the length of the electrode, **characterized in that** the distance between the active contacts (2, 4, 5, 6) is selected to be greater in an apical end region than in a basal region.

2. Multi-channel electrode according to Claim 1, **characterized in that** the region with the larger contact spacing corresponds to an insertion depth of the electrode (3) of greater than 20 mm, preferably 20 to 32 mm.

3. Multi-channel electrode according to Claim 1 or 2, **characterized in that** the contact spacing is increased in at least two spacing levels, whereby in the first subregion corresponding to a central region of the electrode (3) the contact spacing is increased by 25 to 50%, in particular by approximately ⅓, and in the apical end corresponds to 1.5 to 2.5 times, in particular 2 times, of the previously increased contact spacing.

4. Multi-channel electrode according to Claim 1, 2, or 3, **characterized in that** the distance between the contacts in the apical region corresponds to at least 2.5 times the distance between the contacts in the basal region of the electrode (3).

5. Multi-channel electrode according to one of Claims 1 through 4, **characterized in that** the contact spacings from the basal end to the apical end in the transition region between the basal and the central regions initially decrease, and in the central and apical regions increase.

## Revendications

1. Electrode multicanal pour implants cochléaires avec une pluralité de contacts répartis sur la longueur de l'électrode, **caractérisée en ce que** l'écart sélectionné entre les contacts actifs (2, 4, 5, 6) est plus grand au niveau de l'extrémité apicale qu'au niveau du secteur basal.

2. Electrode multicanal selon la revendication 1, **caractérisée en ce que** la zone avec l'écart le plus important entre les contacts correspond à une profondeur d'insertion de l'électrode (3) de plus de 20 mm, de préférence entre 20 et 32 mm.

3. Electrode multicanal selon la revendication 1 ou 2, **caractérisée en ce que** l'écart entre les contacts est agrandi dans au moins deux niveaux d'écart, l'écart entre les contacts étant agrandi de 25 à 50 %, en particulier d'environ 1/3 dans le premier secteur partiel correspondant à un secteur médian de l'électrode (3) et correspondant dans le secteur apical à 1,5 fois à 2,5 fois l'écart, en particulier deux fois, l'écart agrandi précédemment entre les contacts.

4. Electrode multicanal selon la revendication 1, 2 ou 3, **caractérisée en ce que** l'écart entre les contacts dans le secteur apical correspond à au moins 2,5 fois l'écart entre les contacts dans le secteur basal de l'électrode (3).

5. Electrode multicanal selon l'une des revendications 1 à 4, **caractérisée en ce que** les écarts entre les contacts diminuent d'abord de l'extrémité basale à l'extrémité apicale dans la zone de transition entre le secteur basal et médian, puis augmentent dans le secteur médian et apical.
